# DEMANDE DE BREVET EUROPEEN

(11) **EP 0 737 471 A2**
(43) Date de publication de la demande: **16.10.1996**
(21) Numéro de dépôt: 96400513.6
(22) Date de dépôt: 12.03.1996
(51) Int. Cl.: A61K 7/48, A61K 33/06, A61K 33/14, A61K 33/24

(54) **Utilisation d'un sel d'une métal alcalino-terreux comme inhibiteur de TNF-alpha dans une composition unique et composition obtenue**

(30) Priorité: 10.04.1995 FR 9504264; 10.04.1995 FR 9504265; 10.04.1995 FR 9504267; 10.04.1995 FR 9504268; 20.09.1995 FR 9510980
(71) Demandeur: L'OREAL, F-75008 Paris (FR)
(72) Inventeur: Breton, Lionel, F-78000 Versailles (FR); de Lacharriere, Olivier, F-75015 Paris (FR)
(74) Mandataire: Lhoste, Catherine

(57) **Abrégé**

L'invention se rapporte à l'utilisation d'un sel d'un métal alcalino-terreux dans une composition cosmétique, pharmaceutique, vétérinaire et/ou dermatologique, pour traiter notamment les peaux sensibles. Elle se rapporte en outre à l'utilisation d'un sel d'un métal alcalino-terreux pour prévenir et/ou lutter contre la rosacée et/ou les irritations cutanées et/ou les dartres et/ou les érythèmes pudiques et/ou les sensations dysesthésiques et/ou les sensations d'échauffement et/ou les prurits de la peau et/ou des muqueuses. Le sel est en particulier le nitrate ou le chlorure de strontium.

## Description

La présente invention se rapporte à l'utilisation de sels de métaux alcalino-terreux et plus spécialement de sel de strontium dans une composition cosmétique, pharmaceutique, vétérinaire et/ou dermatologique ainsi qu'à la composition obtenue. Plus spécialement, ces sels permettent de traiter, par voie topique, les désordres pathologiques et/ou physiologiques associés à la libération de la substance P et/ou du TNF-alpha (Tumor Necrosis Factor-alpha) et notamment de traiter les peaux sensibles, les désordres et maladies cutanées où existe un prurit, la rosacée et l'érythème pudique.

Il est connu que certaines peaux sont plus sensibles que d'autres. Les symptômes des peaux sensibles étaient jusqu'à présent mal caractérisés et le problème de ces peaux était, de ce fait, mal défini ; personne ne connaissait exactement le processus mis en cause dans la sensibilité -hyperréactivité cutanée non allergique- de la peau. Certains pensaient qu'une peau sensible était une peau qui réagissait aux produits cosmétiques et/ou dermatologiques, d'autres qu'il s'agissait d'une peau qui réagissait à plusieurs facteurs extérieurs, pas forcément liés aux produits cosmétiques et/ou dermatologiques.

Certains tests ont été essayés pour tenter de cerner les peaux sensibles, par exemple des tests à l'acide lactique et au DMSO qui sont connus pour être des substances irritantes : voir par exemple l'article de K. Lammintausta et al., Dermatoses, 1988, 36, pages 45-49; et l'article de T. Agner et J. Serup, Clinical and Experimental Dermatology, 1989, 14, pages 214-217. Mais ces tests ne permettaient pas de caractériser les peaux sensibles.

Par ailleurs, on assimilait les peaux sensibles à des peaux allergiques.

Du fait que l'on connaissait mal les caractéristiques des peaux sensibles, il était jusqu'à présent très difficile de les traiter, et on les traitait indirectement, par exemple en limitant dans les compositions cosmétiques et/ou dermatologiques l'emploi de produits à caractère irritant tels que les tensioactifs, les conservateurs, les parfums ainsi que l'emploi de certains actifs.

La demanderesse a réalisé de nombreux tests cliniques et elle a su déterminer les symptômes liés aux peaux sensibles. Ces symptômes sont en particulier des signes subjectifs, qui sont essentiellement des sensations dysesthésiques. On entend par sensations dysesthésiques des sensations plus ou moins douloureuses ressenties dans une zone cutanée comme les picotements, fourmillements, démangeaisons ou prurits, brûlures, échauffements, inconforts, tiraillements, etc.

La demanderesse a pu montrer en outre qu'une peau sensible n'était pas une peau allergique. En effet, une peau allergique est une peau qui réagit à un agent extérieur, un allergène, qui déclenche une réaction d'allergie. Il s'agit d'un processus immunologique qui ne se produit que lorsqu'un allergène est présent et qui ne touche que les sujets sensibilisés. La caractéristique essentielle de la peau sensible est selon la demanderesse, au contraire, un mécanisme de réponse à des facteurs extérieurs, qui peut concerner tout individu, même si les individus dits à peau sensible y réagissent plus vite que les autres. Ce mécanisme n'est pas immunologique, il est aspécifique.

La demanderesse a maintenant trouvé que les peaux sensibles pouvaient être scindées en deux grandes formes cliniques, les peaux irritables et les peaux intolérantes.

Une peau irritable est une peau qui réagit par un prurit, c'est-à-dire par des démangeaisons ou par des picotements, à différents facteurs tels que l'environnement, les émotions, les aliments, le vent, les frottements, le rasoir, l'eau dure à forte concentration de calcaire, les variations de température ou la laine. En général, ces signes sont associés à une peau sèche avec ou sans dartres, ou à une peau qui présente un érythème.

Une peau intolérante est une peau qui réagit par des sensations d'échauffement, de tiraillements, de fourmillements et/ou de rougeurs, à différents facteurs tels que l'application de produits cosmétiques ou dermatologiques ou de savon. En général, ces signes sont associés un érythème et à une peau hyperséborrhéique ou acnéique, voire même rosacéiforme, avec ou sans dartres.

D'une manière générale, les peaux sensibles se définissent par une réactivité particulière de la peau. Cette hyper-réactivité peut être notamment mise en jeu par des facteurs environnementaux, émotionnels, alimentaires ou encore par l'application ou le contact de produits cosmétiques ou dermatologiques. Cet état d'hyper-réactivité qui définit les peaux sensibles différencie celles-ci de la réactivité ubiquitaire provoquée par des agents irritants qui induisent une irritation de la peau chez la quasitotalité des individus.

Cet état d'hyper-réactivité est ressentie et reconnue par les individus qui en sont atteints comme une « peau sensible ».

Les cuirs chevelus "sensibles" ont une sémiologie clinique plus univoque : les sensations de prurit et/ou de picotements et/ou d'échauffements sont essentiellement déclenchées par des facteurs locaux tels que frottements, savon, tensioactifs, eau dure à forte concentration de calcaire, shampooings ou lotions. Ces sensations sont aussi parfois déclenchées par des facteurs tels que l'environnement, les émotions et/ou les aliments. Un érythème et une hyperséborrhée du cuir chevelu ainsi qu'un état pelliculaire sont fréquemment associés aux signes précédents.

Par ailleurs, dans certaines régions anatomiques comme les grands plis (régions inguinales, génitale, axillaires, poplitées, anale, sous-mammaires, plis du coude) et les pieds, la peau sensible se traduit par des sensations prurigineuses et/ou des sensations dysesthésiques (échauffement, picotements) liées en particulier à la sueur, aux frottements, à la laine, aux tensioactifs, à l'eau dure à forte concentration en calcaire et/ou aux variations de température.

Pour déterminer si une peau est sensible ou non, la demanderesse a également mis au point un test. En effet, après avoir effectué un grand nombre de tests dans le but de définir une peau sensible, elle a trouvé de manière surprenante qu'il existait un lien entre les personnes à peau sensible et celles qui réagissaient à une application topique de capsaïcine.

Le test à la capsaïcine consiste à appliquer sur environ 4 cm² de peau 0,05 ml d'une crème contenant 0,075 % de capsaïcine et à noter l'apparition de signes subjectifs provoqués par cette application, tels que picotements, brûlures et démangeaisons. Chez les sujets à peaux sensibles, ces signes apparaissent entre 3 et 20 minutes après l'application et sont suivis de l'apparition d'un érythème qui débute à la périphérie de la zone d'application.

La capsaïcine était utilisée comme médicament, en particulier pour traiter les douleurs du zona. La capsaïcine provoque un relargage des neuropeptides à partir des fibres nerveuses sensitives, et en particulier des tachykinines comme la substance P et du CGRP (peptide dérivé du gène de la calcitonine : Calcitonin Gene Related Peptide en terminologie anglosaxonne) qui proviennent de terminaisons nerveuses de l'épiderme et du derme. La demanderesse a constaté que le schéma physiopathologique commun à tous les états des peaux sensibles était lié à une grande aptitude à libérer des neuropeptides et plus particulièrement des tachykinines et du CGRP dans la peau. Les manifestations dysesthésiques qui sont provoquées par leur libération sont dites "neurogènes".

On sait, en outre, que les neuropeptides libérés par les terminaisons sensitives épidermiques induisent une cascade d'événements biochimiques dont la libération de médiateurs pro-inflammatoires parmi lesquels le Tumor Necrosis Factor-alpha (TNF-α), l'histamine, l'interleukine 1, l'interleukine 6, l'interleukine 8.

La substance P est un élément chimique polypeptidique élaboré et libéré par une terminaison nerveuse. Elle intervient notamment dans la transmission de la douleur et dans des maladies du système nerveux central telles que l'anxiété, la schizophrénie, dans des maladies respiratoires et inflammatoires, dans des maladies gastrointestinales, dans des maladies rhumatismales et dans certaines maladies dermatologiques telles que l'eczéma, la dermite atopique, le psoriasis et l'acné.

Le CGRP est aussi un élément chimique polypeptidique élaboré et libéré par une terminaison nerveuse. Il intervient notamment dans des maladies respiratoires et inflammatoires, dans des maladies allergiques et rhumastismales et dans certaines maladies dermatologiques telles que l'eczéma, l'urticaire, les dermites de contact ou le prurigo.

La demanderesse a maintenant découvert que l'une des caractéristiques essentielles des peaux sensibles, réactions d'irritation et d'intolérance cutanée, était liée à la libération de neuropeptides et/ou de médiateurs pro-inflammatoires comme le TNF-α et que les molécules qui s'opposaient à la libération de ces neuropeptides et/ou à la libération de TNF-α pouvaient permettre d'obtenir un effet préventif et/ou curatif des peaux sensibles.

Par ailleurs, la demanderesse a trouvé de façon surprenante que les sels de métaux alcalino-terreux étaient des inhibiteurs de TNF-alpha et que certains de ces sels étaient, de plus, des antagonistes de substance P.

Par la demande FR-A-2 719 476 du 5 mai 1994 déposée au nom du demandeur, il est connu d'utiliser des antagonistes de substance P empêchant la synthèse et/ou la libération et/ou la fixation de la substance P pour traiter les peaux sensibles. Toutefois, les sels de métaux alcalino-terreux ne sont pas listés comme antagonistes de substance P, susceptibles de traiter les peaux sensibles.

Pour traiter les peaux sensibles, la demanderesse a donc envisagé d'utiliser des sels de métaux alcalino-terreux notamment comme inhibiteurs de TNF-alpha. Elle a en effet constaté de manière surprenante que l'incorporation d'un sel d'un métal alcalino-terreux dans une composition cosmétique et/ou dermatologique permettait d'éviter l'irritation et/ou les sensations dysesthésiques et/ou les prurits de la peau et/ou des muqueuses notamment en diminuant la libération de neuropeptides dans la peau et/ou les muqueuses.

Personne jusqu'à ce jour n'avait établi un lien entre les sels de métaux alcalino-terreux et la peau sensible. Les signes cliniques de la peau sensible sont essentiellement subjectifs: picotements, fourmillements, prurits, tiraillements, échauffements, et ils s'associent parfois à des érythèmes. Ces signes sont dûs à des facteurs extérieurs aspécifiques. Les symptômes apparaissent essentiellement localisés au visage, au cou et au cuir chevelu, mais peuvent apparaître aussi sur tout le corps.

Le prurit est un symptôme occasionnant une gêne souvent notable pour le patient; lorsque le prurit est très sévère, la gêne peut être telle que le patient ne peut poursuivre son activité habituelle. En outre, le prurit peut être source de complications : excoriations qui peuvent se surinfecter, lichénification des zones prurigineuses, qui ont pour conséquence d'installer la maladie dans un véritable cercle vicieux.

Le prurit est non seulement associé aux peaux sensibles mais aussi à des désordres cutanés comme la dermatite atopique, les dermites de contact, les lichens plans, les prurigos, les urticaires, les toxidermies prurigineuses et certaines formes cliniques de psoriasis.

De plus, le prurit est parfois le signe pathologique cutané prédominant comme dans les cas du prurit aquagénique, du prurit du cuir chevelu lors des états pelliculaires *(pityriasis capitis),* du prurit des hémodialysés, des insuffisants rénaux, des sidéens et des personnes atteintes d'obstructions biliaires, ou encore du prurit des manifestations paranéoplastiques de certains cancers.

Jusqu'à ce jour, les prurits étaient traités à l'aide de préparations émollientes, de corticoïdes locaux, de puvathérapie ou encore d'antihistaminiques. Les corticoïdes locaux sont certes très efficaces pour calmer les symptômes mais malheureusement, leur effet n'est pas immédiat. De plus, ils présentent des effets secondaires souvent très pénalisants comme des atrophies, et exposent à des risques d'infections mycosique et/ou bactérienne et/ou virale. La puvathérapie est l'irradiation locale de la peau malade avec des UVA, après absorption d'une substance photosensibilisante (psoralène). Cette technique présente les inconvénients graves d'un photovieillissement pouvant entraîner des cancers de la peau. De plus, ce traitement n'est pas ambulatoire, obligeant les malades à se rendre couramment dans un centre spécialisé pendant toute la durée du traitement, ce qui est très contraignant et limite leur activité professionnelle.

Les émollients ont un effet anti-prurigineux très modeste et sont peu efficaces lorsque les prurits sont importants. Par ailleurs, les antihistaminiques n'ont pas une efficacité constante et nécessitent une prise orale.

Il subsiste donc le besoin d'un traitement de ces affections prurigineuses, ne présentant pas ces inconvénients.

La présente invention a donc pour objet l'utilisation d'au moins un sel d'un métal alcalino-terreux pour traiter efficacement les prurits, tout en remédiant aux inconvénients mentionnés ci-dessus.

Personne n'avait jusqu'à ce jour envisagé de traiter de façon symptomatique les prurits au moyen de sels d'un métal alcalino-terreux.

Comme autre désordre cutané apparenté aux peaux sensibles, il faut encore citer la rosasée qui est une affection cutanée caractérisée par un érythème du visage prédominant sur les pommettes, le front, le nez, une hyperséborrhée du visage au niveau du front, du nez et des joues, et par une composante infectieuse avec des pustules acnéiformes.

Par ailleurs, il s'associe à ces signes une hyperréactivité cutanée caractérisée par l'apparition de rougeurs et de sensations subjectives du type démangeaisons ou prurit, de sensations de brûlures ou d'échauffement, de sensations de picotements, de fourmillements, d'inconforts, de tiraillements, etc.

Ces signes d'hyperréactivité peuvent être déclenchés par des facteurs très divers tels que la prise d'aliments, de boissons chaudes ou alcoolisées, par des variations de température rapide, par la chaleur et notamment l'exposition aux ultraviolets ou aux infrarouges, par une humidité relative basse, par l'exposition de la peau aux vents violents ou aux courants d'air (soufflerie, air conditionné), par l'application de tensio-actifs ou l'utilisation de certains cosmétiques même lorsque ceux-ci ne sont pas connus comme particulièrement irritants.

La demanderesse a trouvé que ces signes, et notamment l'érythème érythrosique caractéristique de la rosacée, étaient liés à une composante inflammatoire neurogène.

Jusqu'alors, le mécanisme de déclenchement de ces signes était très mal connu et la rosacée était traitée par des actifs tels que les antiséborrhéiques et les anti-infectieux, par exemple le peroxyde de benzoyle, l'acide rétinoïque, le métronidazol ou les cyclines, qui agissaient sur l'infection et l'hyperséborrhée, mais ne permettaient pas de traiter la composante neurogène de cette affection, et notamment l'hyperréactivité de la peau et la rougeur.

De même, il n'existait pas jusqu'à présent de traitement pour la rougeur apparaissant dans l'érythème pudique. Celui-ci survient lors d'une émotion et se caractérise par une rougeur du visage et du décolleté, qui peut éventuellement s'accompagner d'un prurit (démangeaisons). Cette affection est très gênante pour les personnes qui en souffrent, et jusqu'alors on ne pouvait la traiter que par des bêta-bloquants, médicaments puissants utilisés pour traiter l'hypertension et présentant de nombreuses contre-indications.

Il subsiste donc le besoin d'un traitement de la rougeur cutanée et de l'état d'hyperréactivité de la peau atteinte de rosacée ou d'érythème pudique.

La présente invention a justement pour objet l'utilisation d'un ou plusieurs sels de métal alcalino-terreux pour traiter ces rougeurs cutanées.

Personne n'avait envisagé jusqu'à ce jour d'utiliser des sels de métal alcalino-terreux pour traiter la rougeur cutanée d'origine neurogène.

La présente invention a donc pour objet l'utilisation d'au moins un sel d'au moins un métal alcalino-terreux dans ou pour la fabrication d'une composition contenant un milieu cosmétiquement, pharmaceutiquement et/ou dermatologiquement acceptable, pour traiter les peaux sensibles humaines et/ou les rougeurs cutanées d'origine neurogène d'humains, rougeurs plus spécialement dues à la libération de tachykinines.

L'application de compositions contenant un ou des sels de métal alcalino-terreux permet d'obtenir une nette diminution voire une disparition complète de la rougeur qui se manifeste aussi bien dans la rosacée que dans l'érythème pudique.

Le sel de métal alcalino-terreux agit donc sur la composante neurogène de ces affections, pour laquelle on n'avait pas de traitement jusqu'à présent, et renforce ainsi l'efficacité des actifs utilisés jusqu'à présent pour le traitement de leur composante infectieuse, notamment dans le cas de la rosacée.

La présente invention a encore pour objet l'utilisation d'au moins un sel d'au moins un métal alcalino-terreux pour prévenir et/ou lutter contre la rosasée, et/ou l'érythème pudique, les irritations cutanées et/ou les dartres et/ou les érythèmes et/ou les sensations d'échauffement et/ou les sensations dysesthésiques et/ou les prurits de la peau et/ou des muqueuses d'humains.

Par prurit, il faut comprendre les symptômes prurigineux des désordres et/ou maladies de la peau ou des muqueuses. Les sels de l'invention sont destinés au traitement des prurits et non au traitement des désordres et/ou maladies responsables de ces prurits.

La présente invention a donc encore pour objet l'utilisation d'au moins un sel d'un métal alcalino-terreux dans ou pour la préparation d'une composition cosmétique, pharmaceutique ou dermatologique pour traiter les symptômes prurigineux des désordres et/ou maladies de la peau.

L'invention a encore pour objet l'utilisation d'au moins un sel d'au moins un métal alcalino-terreux comme inhibiteur de TNF-alpha, dans ou pour la fabrication d'une composition contenant un milieu cosmétique, pharmaceutique, dermatologique ou vétérinaire.

L'invention a encore pour objet l'utilisation d'au moins un sel d'au moins un métal alcalino-terreux comme inhibiteur de TNF-alpha, dans ou pour la fabrication d'une composition contenant un milieu cosmétiquement, pharmaceutiquement ou dermatologiquement acceptable, pour traiter les peaux sensibles.

L'invention a encore pour objet l'utilisation d'au moins un sel d'au moins un métal alcalino-terreux comme inhibiteur de TNF-alpha, dans ou pour la fabrication d'une composition cosmétique, pharmaceutique, dermatologique ou vétérinaire destinée au traitement des désordres pathologiques et/ou physiologiques associés à la libération de TNF-alpha.

Pour qu'une substance soit reconnue comme un inhibiteur de TNF-alpha, elle doit répondre notamment à l'une au moins des caractéristiques suivantes :
- inhibition de la libération du TNF-alpha par des monocytes (cellules U937) stimulés par un ester de phorbol (PMA)
- inhibition de la fixation du TNF-alpha sur son récepteur membranaire.

Les inhibiteurs de TNF-alpha selon l'invention peuvent, en outre, être utilisés pour traiter notamment la fièvre, le choc septique et/ou la cachexie.

Un milieu cosmétiquement, physiologiquement, pharmaceutiquement et/ou dermatologiquement acceptable est un milieu qui est compatible avec la peau, le cuir chevelu, les ongles et les muqueuses des humains. La composition contenant un ou plusieurs sels d'un ou plusieurs métaux alcalino-terreux peut donc être appliquée sur le visage, le cou, les cheveux et les ongles, ou toute autre zone cutanée du corps humain comme les grands plis (régions axillaires, sous-mammaires, plis du coude, etc.), les muqueuses (anale, génitale, nasale).

Un milieu vétérinaire, voire physiologique est un milieu compatible avec la peau, les griffes et les muqueuses des animaux.

Selon l'invention, on peut utiliser un ou plusieurs sels de métaux alcalino terreux. Comme sels utilisables dans l'invention, on peut citer les sels de baryum, de calcium, de magnésium, de strontium et/ou de béryllium. Les sels de l'invention peuvent être anhydres ou hydratés.

Ces sels peuvent être par exemple des carbonates, des bicarbonates, des sulfates, des glycérophosphates, des borates, des chlorures, des nitrates, des acétates, des hydroxydes, des persulfates ainsi que des sels d'α-hydroxyacides (citrates, tartrates, lactates, malates) ou d'acides de fruits, ou encore des sels d'acides aminés (aspartate, arginate, glycocholate, fumarate) ou des sels d'acides gras (palmitate, oléate, caséinate, béhénate). De préférence le sel est choisi parmi le nitrate, le borate, le chlorure, le sulfate et l'acétate de calcium, de strontium ou de magnésium.

De façon encore plus avantageuse, le sel est un sel de magnésium et mieux de strontium se présentant notamment sous forme de chlorure ou de nitrate.

Les sels de strontium et à un niveau moindre de magnésium sont des antagonistes de substances P.

Pour qu'une substance soit reconnue comme un antagoniste de substance P, elle doit répondre notamment à la caractéristique suivante :
- avoir une activité pharmacologique antagoniste de la substance P, c'est-à-dire induire une réponse pharmacologique cohérente dans au moins l'un des deux tests suivants :
   - la substance antagoniste doit diminuer l'extravasation du plasma au travers de la paroi vasculaire induite par la capsaïcine ou par une stimulation nerveuse antidromique, ou bien
   - la substance antagoniste doit provoquer une inhibition de la contraction des muscles lisses induites par l'administration de substance P.

Par ailleurs, la substance antagoniste de substance P peut avoir une affinité sélective pour les récepteurs NK1 des tachykinines.

La demanderesse a constaté de manière surprenante que les sels de strontium présentaient la caractéristique d'inhiber la fixation réceptorielle de substance P et pouvaient donc être utilisés comme antagonistes réceptoriel de substance.

Aussi, l'invention a encore pour objet l'utilisation d'au moins un sel de strontium dans un milieu physiologiquement acceptable dans et/ou pour la fabrication d'une composition cosmétique, dermatologique, pharmaceutique et/ou vétérinaire destinée à traiter les désordres pathologiques et/ou physiologiques associés à la libération de substance P.

Les désordres physiologiques associés à la substance P sont notamment les affections cutanées (dermatite atopique, psoriasis, eczéma, acné, urticaire, névrodermite. prurigo, peaux sensibles, prurits), les affections bronchopulmonaires (asthme, bronchite allergique, rhinite allergique), les algies (algies dentaires, ophtalmiques, tympaniques, rhumatismales, post-traumatiques, et migraines), les affections gastro-intestinales, les maladies du système nerveux central (dépression, schizophrénie).

Aussi, l'invention a encore pour objet l'utilisation d'au moins un sel de strontium dans un milieu physiologiquement acceptable dans et/ou pour la fabrication d'une composition cosmétique, dermatologique, pharmaceutique et/ou vétérinaire destinée à traiter les affections cutanées, les affections bronchopulmonaires, les algies, les affections gastro-intestinales et/ou les maladies du système nerveux central.

L'invention a encore pour objet l'utilisation d'au moins un sel de strontium dans un milieu physiologiquement acceptable dans et/ou pour la fabrication d'une composition cosmétique, dermatologique, pharmaceutique et/ou vétérinaire destinée à traiter la dermatite atopique, l'eczéma, le psoriasis, l'acné.

Dans les compositions selon l'invention, le sel de métal alcalino-terreux est utilisé de préférence en une quantité représentant de 0,001 à 30 % du poids total de la composition, et en particulier en une quantité représentant de 0,01 à 20 % du poids total de la composition et mieux de 0,5 à 10 %.

L'invention a encore pour objet un procédé cosmétique, pharmaceutique ou vétérinaire pour inhiber la fixation et/ou la synthèse et/ou la libération de TNF-alpha dans la peau, caractérisé en ce qu'on applique sur la peau, sur le cuir chevelu et/ou sur les muqueuses, une composition contenant au moins un sel d'au moins un métal alcalino-terreux dans un milieu cosmétique, pharmaceutique ou vétérinaire.

La présente invention a en outre pour objet un procédé de traitement cosmétique et/ou dermatologique des peaux sensibles et/ou des rougeurs cutanées d'origine neurogène, caractérisé par le fait que l'on applique sur la peau, sur le cuir chevelu et/ou sur les muqueuses sensibles et/ou rosacés, une composition telle que décrite ci-dessus contenant au moins un sel d'au moins un métal alcalino-terreux dans un milieu cosmétiquement et/ou dermatologiquement acceptable.

La présente invention a en outre pour objet un procédé de traitement cosmétique et/ou dermatologique des symptômes prurigineux des désordres et/ou maladies de la peau et/ou des muqueuses, caractérisé par le fait que l'on applique sur la peau, sur le cuir chevelu et/ou sur les muqueuses, une composition telle que décrite ci-dessus contenant au moins un sel d'au moins un métal alcalino-terreux dans un milieu cosmétiquement et/ou dermatologiquement acceptable.

L'invention a aussi pour objet un procédé de traitement cosmétique et/ou dermatologique des désordres physiologiques associés à la libération de substance P, caractérisé en ce qu'on applique sur la peau, le cuir chevelu et/ou les muqueuses une composition contenant au moins un sel de strontium tel que défini ci-dessus dans un milieu cosmétiquement et/ou dermatologiquement acceptable.

De façon avantageuse, on associe aux sels de métaux alcalino-terreux un ou plusieurs antagonistes d'un ou plusieurs neuropeptides, ces antagonistes étant de préférence réceptoriels, et/ou un ou plusieurs antagonistes d'un ou plusieurs médiateurs de l'inflammation.

Aussi, l'invention a encore pour objet une composition cosmétique, pharmaceutique, vétérinaire et/ou dermatologique contenant, dans un milieu cosmétique, pharmaceutique, vétérinaire et/ou dermatologique, au moins un sel d'au moins un métal alcalino-terreux et au moins un antagoniste de neuropeptide et/ou au moins un antagoniste de médiateur de l'inflammation, différents desdits sels, notamment pour traiter les peaux sensibles.

Comme antagonistes de neuropeptides utilisables dans l'invention, on peut citer les antagonistes de substance P et les antagonistes de CGRP et comme antagonistes de médiateurs de l'inflammation, on peut citer les antagonistes d'histamine, d'interleukine 1 ou de TNFα. Ces antagonistes peuvent être présents à raison de 0,000001 à 10 % du poids total de la composition et mieux de 0,0001 à 5 %.

De façon avantageuse, on utilise des antagonistes de préférence réceptoriels de substance P, de CGRP et/ou d'interleukine 1.

Comme antagoniste de substance P utilisable dans l'invention, on peut citer toute substance d'origine organique ou minérale, capable de produire une inhibition de la fixation réceptorielle de substance P ou une inhibition de la synthèse et/ou la libération de substance P par des fibres nerveuses sensitives.

L'antagoniste réceptoriel de substance P peut être notamment un peptide ou un dérivé non peptidique comportant un hétéroatome, et plus précisément un composé comportant un hétérocycle ou un hétéroatome lié directement ou indirectement à un cycle benzénique.

On peut utiliser par exemple comme peptide antagoniste de substance P réceptoriel le sendide et le spantide Il.

On peut également utiliser dans l'invention comme peptide ceux décrits dans les documents US-A-4472305, US-A-4839465, EP-A-101929, EP-A-333174, EP-A-336230, EP-A-394989, EP-A-443132, EP-A-498069, EP-A-515681, EP-A-517589, WO-A-92/22569 et GB-A-2216529.

Les antagonistes réceptoriels de substance P non peptidiques utilisables dans l'invention sont notamment des composés hétérocycliques notamment soufrés, azotés ou oxygénés ou des composés comprenant un atome d'azote lié directement ou indirectement à un cycle benzénique.

Comme composé hétérocyclique, on peut utiliser dans l'invention ceux décrits dans les documents suivants : EP-A-360390, EP-A-429366, EP-A-430771, EP-A-499313, EP-A-514273, EP-A-514274, EP-A-514275, EP-A-514276, EP-A-520555, EP-A- 528495, EP-A-532456, EP-A-545478, EP-A-558156, WO-A-90/05525, WO-A-90/05729, WO-A-91/18878, WO-A-91/18899, WO-A-92/12151 , WO-A-92/15585, WO-A-92/17449, WO-A-92/20676, WO-A-93/00330, WO-A-93/00331, WO-A-93/01159, WO-A-93/01169, W0-A-93/01170, WO-A-93/06099, WO-A-93/09116 et WO-A-94/08997. En particulier, le composé comprenant au moins un hétérocycle azoté est un dérivé de 2-tricyclyl-2-amino-éthane, un dérivé de spirolactame, un dérivé de quinuclidine, un dérivé azacyclique, un dérivé d'aminopyrrolidine, un dérivé de pipéridine, un aminoazahétérocycle ou un dérivé d'isoindole.

Comme composés comportant un atome d'azote lié directement ou indirectement à un noyau benzénique, on peut citer ceux décrits dans les documents suivants : EP-A-522808 et WO-A-93/01165.

Comme antagoniste de CGRP utilisable dans l'invention, on peut citer toute substance d'origine organique ou minérale capable de produire une inhibition de la fixation réceptorielle du CGRP ou de produire une inhibition de la synthèse et/ou de la libération de CGRP par les fibres nerveuses sensitives.

Pour qu'une substance soit reconnue comme un antagoniste de CGRP, elle doit répondre notamment à la caractéristique suivante :
- avoir une activité pharmacologique antagoniste du CGRP, c'est-à-dire induire une réponse pharmacologique cohérente notamment dans l'un des tests suivants:
   - la substance antagoniste doit diminuer la vasodilation induite par la capsaïcine et/ou
   - la substance antagoniste doit provoquer une inhibition de la libération de CGRP par les fibres nerveuses sensitives et/ou
   - la substance antagoniste doit provoquer une inhibition de la contraction du muscle lisse du canal déférent induite par le CGRP.

En outre, l'antagoniste de CGRP peut avoir une affinité pour les récepteurs au CGRP.

On peut utiliser dans l'invention par exemple comme antagoniste réceptoriel de CGRP, le CGRP 8-37, un anticorps anti-CGRP.

Les compositions selon l'invention peuvent être appliquées soit par voie locale, c'est-à-dire par voie topique ou par injection sous-cutanée et/ou intradermique, soit par voie systémique ou générale, c'est-à-dire par voie orale et/ou injection intramusculaire.

Les compositions selon l'invention peuvent se présenter sous toutes les formes galéniques normalement utilisées selon la voie d'utilisation (application topique, injectable ou ingérable).

Les compositions injectables peuvent se présenter sous forme d'une lotion aqueuse, huileuse ou sous forme de sérum.

Les compositions utilisées par voie orale peuvent se présenter sous forme de capsules, de gélules, de sirops ou de comprimés.

Pour une application topique, les compositions peuvent se présenter notamment sous forme de solutions aqueuses, hydroalcooliques ou huileuses, de dispersions du type des solutions ou dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème, de gel aqueux ou anhydre, ou encore de microémulsions, de microcapsules, de microparticules, ou de dispersions vésiculaires de type ionique et/ou non ionique.

Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol contenant également un agent propulseur sous pression.

Les quantités des différents constituants des compositions selon l'invention sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des produits de maquillage comme des fonds de teint fluides, des laits de démaquillage, des laits corporels de protection ou de soin, des laits après-solaires, des lotions, gels ou mousses pour le soin de la peau, comme des lotions de nettoyage ou de désinfection, des lotions anti-solaires, des lotions de bronzage artificiel, des compositions pour le bain, des compositions déodorantes contenant un agent bactéricide, des gels ou lotions après-rasage, des crèmes épilatoires, des compositions contre les piqûres d'insectes, des compositions anti-douleur ou des compositions pour traiter certaines maladies de la peau comme les prurits sévères, la rosacée, l'acné, les ulcères de la jambe, le psoriasis, les pustules, les vergetures.

Les compositions selon l'invention peuvent également consister en des préparations solides constituant des savons ou des pains de nettoyage.

Le sel d'un métal alcalino-terreux peut être aussi incorporé dans diverses compositions pour soins capillaires, et notamment des shampooings éventuellement antiparasitaires, des lotions de mise en plis, des lotions traitantes, des crèmes ou des gels coiffants, des compositions de teintures (notamment teintures d'oxydation) éventuellement sous forme de shampooings colorants, des lotions restructurantes pour les cheveux, des compositions de permanente (notamment des compositions pour le premier temps d'une permanente), des lotions ou des gels antichute, etc.

Les compositions de l'invention peuvent aussi être à usage bucco-dentaire, par exemple une pâte dentifrice ou un bain de bouche. Dans ce cas, les compositions peuvent contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Lorsque la composition de l'invention est une émulsion, la proportion de la phase grasse peut aller de 5 % à 80 % en poids, et de préférence de 5 % à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique et/ou dermatologique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition de l'invention est une solution ou un gel huileux, la phase grasse peut représenter plus de 90 % du poids total de la composition.

De façon connue, la composition cosmétique et/ou dermatologique de l'invention peut contenir également des adjuvants habituels dans le domaine cosmétique pharmaceutique et/ou dermatologique, tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur et les matières colorantes. Les quantités de ces différents adjuvants sont celles classiquement utilisées dans le domaine considéré, et par exemple de 0,01 % à 20 % du poids total de la composition. Ces adjuvants, selon leur nature, peuvent être introduits dans la phase grasse, dans la phase aqueuse et/ou dans les sphérules lipidiques.

Comme matières grasses utilisables dans l'invention, on peut citer les huiles minérales (polyisobutène hydrogéné, huile de vaseline), les huiles végétales (fraction liquide du beurre de karité, huile de tournesol, d'amandes d'abricot), les huiles animales (perhydrosqualène), les huiles de synthèse (huile de Purcellin, myristate d'isopropyle, palmitate d'éthyl hexyle), et les huiles fluorées (perfluoropolyéthers). On peut aussi utiliser des alcools gras, des acides gras (acide stéarique), des cires (paraffine, carnauba, cire d'abeilles).

On peut aussi utiliser des composés siliconés comme les huiles siliconées (cyclométhicone, diméthicone), les cires, les résines et les gommes. Ces composés peuvent être fonctionnalisés ou non.

Comme émulsionnants utilisables dans l'invention, on peut citer par exemple le stéarate de glycérol, le polysorbate 60, le mélange alcool cétylstéarylique/alcool cétylstéarylique oxyéthyléné à 33 moles d'oxyde d'éthylène (Sinnowax AO vendu par la société Henkel), le mélange de PEG-6/PEG-32/Glycol Stéarate vendu sous la dénomination de Tefose^{R} 63 par la société Gattefosse, le PPG-3 myristyl éther, les émulsionnants siliconés tels que le cétyldiméthicone copolyol et le mono- ou tristéarate de sorbitane, le stéarate de PEG-40, le monostéarate de sorbitane oxyéthyléné (20OE).

Comme solvants utilisables dans l'invention, on peut citer les alcools inférieurs, notamment l'éthanol et l'isopropanol, le propylène glycol.

Comme gélifiants hydrophiles, on peut citer les polymères carboxyvinyliques (carbomer), les copolymères acryliques tels que les copolymères d'acrylates/alkylacrylates, les polyacrylamides et notamment le mélange de polyacrylamide, C13-14-Isoparaffine et Laureth-7 vendu sous le nom de Sepigel 305 par la société Seppic, les polysaccharides (dérivés cellulosiques) tels que les hydroxyalkylcelluloses (hydroxypropylcellulose, hydroxyéthylcellulose), gommes naturelles (guar, caroube, xanthane) et argiles, et, comme gélifiants lipophiles, on peut citer les argiles modifiées comme les bentones, les sels métalliques d'acides gras comme les stéarates d'aluminium et la silice hydrophobe, ou encore l'éthylcellulose (dérivés cellulosiques), le polyéthylène.

Comme actifs hydrophiles, on peut utiliser les protéines ou les hydrolysats de protéine, les acides aminés, les polyols notamment en C₂ à C₁₀ (glycérine, sorbitol, butylène glycol, polyéthylène glycol), l'urée, l'allantoïne, les sucres et les dérivés de sucre, les vitamines hydrosolubles, l'amidon, des extraits bactériens ou végétaux comme ceux d'Aloe Vera.

Comme actifs lipophiles, on peut utiliser le rétinol (vitamine A) et ses dérivés, le tocophérol (vitamine E) et ses dérivés, les acides gras essentiels, les céramides, les huiles essentielles.

On peut, entre autre, associer les sels de métaux alcalino-terreux à des agents actifs destinés notamment à la prévention et/ou au traitement des affections cutanées.

Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, le rétinal, les rétinoïdes, notamment ceux décrits dans les documents FR-A-2 570 377, EP-A-199636, EP-A-325540, EP-A-402072, la vitamine D et ses dérivés, les estrogènes tels que l'estradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyénes, tels que l'amphotéricine B, les composés de la famille des allylamines, tels que la terbinafine, ou encore l'octopirox ;
- les agents anti-inflammatoires stéroïdiens, tels que l'hydrocortisone, les anthralines (dioxyanthranol), les anthranoïdes, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine;
- les agents antiviraux tels que l'acyclovir;
- les agents kératolytiques tels que les acides alpha- et bêta-hydroxy-carboxyliques ou bêta-cétocarboxyliques, leurs sels, amides ou esters et plus particulièrement les hydroxyacides tels que l'acide glycolique, l'acide lactique, l'acide malique, l'acide salicylique, l'acide citrique et de manière générale les acides de fruits, et l'acide n-octanoyl-5-salicylique;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle ;
- les antimétabolites ;
- les agents pour lutter contre la chute des cheveux comme le monoxidil ;
- les antiseptiques
- les corticoïdes.

Il est connu que les corticoïdes sont efficaces pour le traitement de l'eczéma, mais que leur utilisation entraîne souvent des effets secondaires indésirables. Or, l'association avec le sel de strontium utilisé selon l'invention permet de diminuer voire supprimer le taux de corticoïde tout en permettant une bonne efficacité sur l'eczéma.

En outre, la composition de l'invention peut contenir de façon avantageuse une eau thermale et/ou minérale, notamment choisie parmi l'eau de Vittel, les eaux du bassin de Vichy, l'eau d'Uriage, l'eau de la Roche Posay, l'eau de la Bourboule, l'eau d'Enghien-les-bains, l'eau de Saint-Gervais-les bains, l'eau de Néris-les-bains, l'eau d'Allevard-les-bains, l'eau de Digne, l'eau des Maizières, l'eau de Neyrac-les-bains, l'eau de Lons le Saunier, les Eaux Bonnes, l'eau de Rochefort, l'eau de Saint Christau, l'eau des Fumades et l'eau de Tercis-les-bains. Il s'agit de préférence de l'eau de la Roche Posay.

Le procédé de traitement cosmétique et/ou dermatologique de l'invention peut être mis en oeuvre notamment en appliquant les compositions hygiéniques ou cosmétiques et/ou dermatologiques telles que définies ci-dessus, selon la technique d'utilisation habituelle de ces compositions. Par exemple : application de crèmes, de gels, de sérums, de lotions, de laits de démaquillage ou de compositions aprè-solaires sur la peau ou sur les cheveux secs, application d'une lotion pour cheveux sur cheveux mouillés, de shampooings, ou encore application de dentifrice sur les gencives.

Des tests ont mis en évidence l'activité de différents sels de strontium comme antagonistes de substance P en référence au Spantide II, connu comme antagoniste de substance P, et comparativement à un autre sel de métal alcalino-terreux, le nitrate de calcium.

Le test a consisté à déterminer l'activité des différents composés sur la libération de substance P, provoquée par stimulation antidromique du nerf sciatique. Cette libération est visualisée par coloration avec un colorant (bleu Evans). La coloration est d'autant plus forte que la quantité de substance P libérée est importante. Autrement dit, plus l'antagoniste de substance P produit un effet inhibiteur sur cette libération, plus la coloration est faible.

Les résultats sont rassemblés dans les tableaux 1 et 2 ci-dessous :

**Tableau 1**

| | Témoin véhicule | Spantide II 30 nmoles | Sr(NO₃)₂ 1 µmole | SrCl₂ 1 µmole | Ca(NO₃)₂ 1 µmole |
|---|---|---|---|---|---|
| µg/ml de bleu Evans | 8,19±0,83 | 4,35±0,46 | 4,84±0,6 | 5,13±0,9 | 11,25±1,77 |
| % d'inhibition par rapport au témoin | - | 47 % | 41 % | 37 % | - |
| Statistiques | - | p<0,01 | p<0,05 | p<0,05 | non significatif |

II ressort de ce tableau 1 que les sels de strontium ont une activité importante d'inhibition de la libération de la substance P alors que le sel de calcium, à taux de concentration égale, n'a pas d'activité du tout.

**Tableau 2**

| | Témoin véhicule | Spantide II 30 nmoles | Sr(NO₃)₂ 3 µmoles | SrCl₂ 1 µmole | Ca(NO₃)₂ 10 µmoles |
|---|---|---|---|---|---|
| µg/ml de bleu Evans | 6,26±1,09 | 3,63±0,69 | 1,80±0,68 | 2,71±0,67 | 8,45±1,55 |
| % d'inhibition par rapport au témoin | - | 42 % | 71 % | 57 % | - |
| Statisti-ques | - | p<0,05 | p<0,001 | p<0,01 | non significatif |

II ressort de ce tableau 2 que les sels de strontium ont une activité importante d'inhibition de la libération de la substance P alors que le sel de calcium n'a pas d'activité du tout, même à un taux de concentration plus important.

Le test suivant permet de montrer que les sels d'alcalino-terreux et plus spécialement le chlorure de magnésium, de strontium ou de calcium ainsi que le nitrate de strontium sont des inhibiteurs de TNF-alpha.

Ce test est réalisé sur des monocytes humains (lignée U937) stimulés par un ester de phorbol (PMA) selon la méthode décrite par Schindler et al (Correlations and interactions in the production of interleukin-6 (IL-6), IL-1, and Tumor Necrosis Factor (TNF) in human blood mononuclear cells : IL-6 suppresses IL-1 and TNF. Blood 75 : 40-47 (1990)).

L'ester de phorbol (PMA) stimule naturellement la synthèse et/ou la sécrétion de TNF-alpha par des monocytes humains en culture.

L'activité des molécules est évaluée en fonction de leurs aptitudes à diminuer, voire à supprimer cette sécrétion de TNF-alpha.

Les cellules (monocytes) sont incubées en présence de PMA à la concentration de 10 nM pendant 48 heures à 37 °C. Les quantités de TNF-alpha sécrétées sont quantifiées par dosage immuno-enzymatique (ECA) à l'aide de kits disponibles dans le commerce.

Chaque molécule est testée à 3 concentrations différentes (10⁻⁵, 10⁻⁴, et 10⁻³ M) et à chaque expérimentation une molécule de référence (dexaméthasone) est étudiée à 7 concentrations comme standard interne.

Les résultats sont exprimés en pourcentage d'inhibition par rapport au témoin positif (sans molécule à tester) après soustraction du bruit de fond. Un tableau 3 synthétique regroupe les effets inhibiteurs moyennés, obtenus avec les composés. (Les résultats sont exprimés en % d'inhibition ; ils sont la moyenne de 3 mesures)

La valeur d'IC₅₀ (diminution de 50 % de la sécrétion, provoquée par le PMA) est calculée, pour la molécule de référence, à partir de la courbe de compétition selon un modèle de régression non linéaire.

**Tableau 3**

| test | composés | 10⁻⁵ M | 10⁻⁴ M | 10⁻³ M | référence | IC₅₀ |
|---|---|---|---|---|---|---|
| % d'inhibition de sécrétion de TNF- alpha induite par le PMA | SrCl₂7H2O | 14 | - | 54 | | |
| | MgCl₂ | - | 12 | 70 | | |
| | | | | | dexaméthasone | 4x10⁻⁹M |
| | | | | | | |
| | CaCl₂,7H₂O | 19 | 22 | 67 | | |
| | Sr(NO₃)₂ | - | 28 | 75 | | |

On constate que l'inhibition de TNF-alpha augmente avec la concentration en sel.

Les exemples suivants illustrent des compositions à application topique conformes à l'invention. Dans ces exemples, les proportions indiquées sont en poids.

### Exemple 1 : Lotion démaquillante pour le visage des peaux sensibles

| | |
|---|---|
| Chlorure de magnésium | 3,00 |
| Antioxydant | 0,05 |
| Isopropanol | 40,00 |
| Conservateur | 0,30 |
| Eau | qsp 100 % |

### Exemple 2 : Lotion démaquillante pour le visage des peaux sensibles

| | |
|---|---|
| CGRP 8-37 | 0,10 |
| Nitrate de calcium | 2,00 |
| Antioxydant | 0,05 |
| Isopropanol | 40,00 |
| Conservateur | 0,30 |
| Eau | qsp 100 % |

### Exemple 3 : Lait pour le soin du visage des peaux sensibles

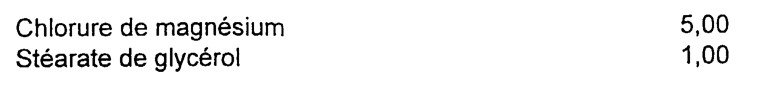

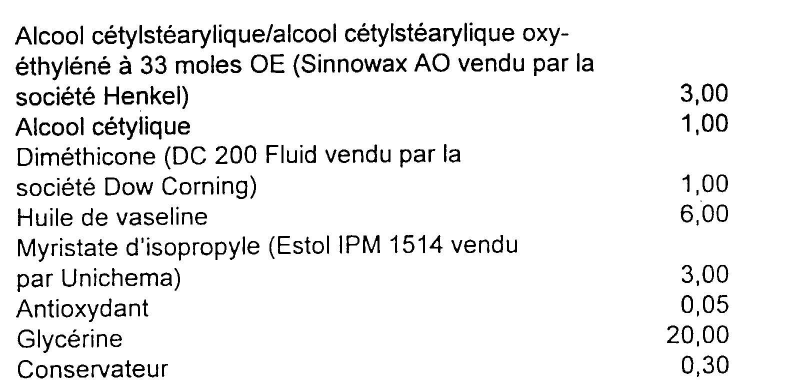

### Exemple 4 : Gel pour le soin du visage des peaux sensibles

| | |
|---|---|
| Nitrate de baryum | 4,00 |
| Hydroxypropylcellulose (Klucel H vendu par la société Hercules) | 1,00 |
| Spantide Il | 0,50 |
| CGRP 8-37 | 0,05 |
| Antioxydant | 0,05 |
| Isopropanol | 40,00 |
| Conservateur | 0,30 |
| Eau | qsp 100 % |

### Exemple 5 : Gel pour le soin du visage des peaux sensibles

| | |
|---|---|
| Nitrate de magnésium | 5,00 |
| Hydroxypropylcellulose (Klucel H de chez Hercules) | 1,00 |
| Antioxydant | 0,05 |
| Isopropanol | 40,00 |
| Conservateur | 0,30 |
| Eau | qsp 100 % |

### Exemple 6 : Shampooing du cuir chevelu sensible

| | |
|---|---|
| Lauryl éther sulfate de sodium (2,2OE) | 12,00 |
| Chlorure de strontium | 5,00 |
| Hydroxypropylcellulose (Klucel H de chez Hercules) | 1,00 |
| Parfum | 0,50 |
| Conservateur | 0,30 |
| Eau | qsp 100 % |

### Exemple 7 : Emulsion H/E pour le soin des peaux sensibles contre les piqûres d'insectes ou de la rosacée

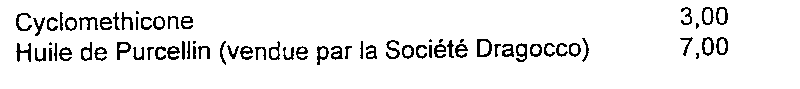

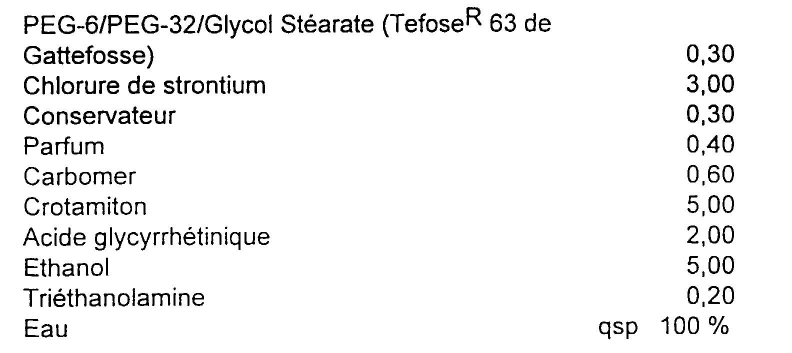

### Exemple 8 : Gel anti-douleur

| | |
|---|---|
| Nitrate de strontium | 3,00 |
| Hydroxypropylcellulose (Klucel H de chez Hercules) | 1,00 |
| Antioxydant | 0,05 |
| Chlorhydrate de lidocaïne | 2,00 |
| Isopropanol | 40,00 |
| Conservateur | 0,30 |
| Eau | qsp 100 % |

### Exemple 9 : Emulsion H/E pour le soin pour le visage des peaux sensibles

| | |
|---|---|
| Nitrate de strontium | 0,25 |
| Stéarate de glycérol | 2,00 |
| Polysorbate 60 (Tween 60 vendu par la société ICI) | 1,00 |
| Acide stéarique | 1,40 |
| Métronidazole | 1,00 |
| Triéthanolamine | 0,70 |
| Sendide | 0,50 |
| Carbomer | 0,40 |
| Fraction liquide du beurre de karité | 12,00 |
| Huile de vaseline | 12,00 |
| Antioxydant | 0,05 |
| Parfum | 0,5 |
| Conservateur | 0,30 |
| Eau | qsp 100 % |

### Exemple 10 : Fluide de soin de l'érythème solaire des peaux sensibles (émulsion huile dans eau)

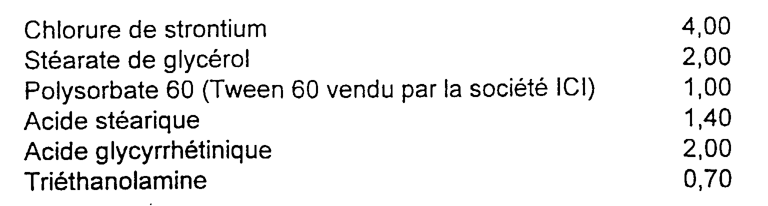

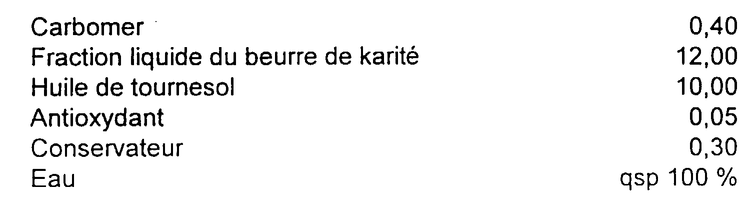

### Exemple 11 : Lotion traitante anti-prurigineuse pour les muqueuses génitales

| | |
|---|---|
| Chlorure de strontium | 6 |
| Antioxydant | 0,05 |
| Isopropanol | 40 |
| Conservateur | 0,3 |
| Eau | qsp 100 % |

### Exemple 12 : Gel pour le traitement du prurit modéré

| | |
|---|---|
| Chlorure de strontium | 4 |
| Hydroxypropylcellulose (Klucel H vendu par la société Hercules) | 1 |
| Antioxydant | 0,05 |
| Isopropanol | 40 |
| Conservateur | 0,3 |
| Eau | qsp 100 % |

### Exemple 13 : Gel corporel pour le traitement du prurit sévère en cours de dermatose

| | |
|---|---|
| Sendide | 0,2 |
| Nitrate de strontium | 5 |
| Hydroxypropylcellulose (Klucel H) | 1 |
| Antioxydant | 0,05 |
| Isopropanol | 40 |
| Conservateur | 0,3 |
| Eau | qsp 100 % |

### Exemple 14 : Crème de soin de la rosacée du visage (émulsion huile dans eau)

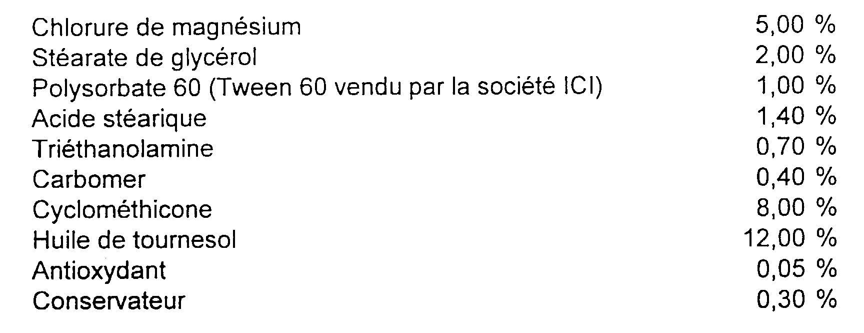

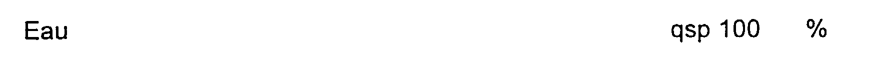

### Exemple 15 : Gel pour rosacée

| | |
|---|---|
| Acide all trans rétinoïque | 0,05 % |
| Sulfate de calcium | 5,00 % |
| Hydroxypropylcellulose (Klucel H vendu par la société Hercules) | 1,00 % |
| Antioxydant | 0,05 % |
| Isopropanol | 40,00 % |
| Conservateur | 0,30 % |
| Eau | qsp 100 % |

### Exemple 16 : Gel pour érythème pudique

L'exemple 16 est identique à l'exemple 15, à l'exception du fait qu'il contient en plus 0,3 % de sendide.

### Exemple 17: Crème pour la rosacée (émulsion huile dans eau)

| | |
|---|---|
| Sulfate de magnésium | 3,00 % |
| Stéarate de glycérol | 2,00 % |
| Polysorbate 60 (Tween 60 vendu par la société ICI) | 1,00 % |
| Acide stéarique | 1,40 % |
| Métronidazole | 0,50 % |
| Triéthanolamine | 0,70 % |
| Carbomer | 0,40 % |
| Cyclométhicone | 8,00 % |
| Huile de tournesol | 10,00 % |
| Antioxydant | 0,05 % |
| Conservateur | 0,30 % |
| Eau | qsp 100 % |

### Exemple 18 : Crème pour la rosacée (émulsion huile dans eau)

| | |
|---|---|
| Chlorure de magnésium | 4,00 % |
| Stéarate de glycérol | 2,00 % |
| Spantide | 0,50 % |
| CGRP 8-37 | 0,25 % |
| Polysorbate 60 (Tween 60 vendu par la société ICI) | 1,00 % |
| Acide stéarique | 1,40 % |
| Triéthanolamine | 0,70 % |
| Carbomer | 0,40 % |
| Cyclométhicone | 8,00 % |
| Huile de tournesol | 12,00 % |
| Antioxydant | 0,05 % |
| Conservateur | 0,30 % |
| Eau | qsp 100 % |

### Exemple 19 : Crème de soin pour dermatite atopique en dehors des poussées eczémateuses

| | |
|---|---|
| - Palmitate d'éthyl hexyle | 3,00% |
| - Fraction liquide de beurre de karité (Triglycérides d'acide palmitique-stéarique-oléïque-linoléïque) | 2,00% |
| - Stéarate de glycérol | 3,00% |
| - Tristéarate de sorbitane (Span 65 de chez ICI) | 0,90% |
| - Polyisobutène hydrogéné | 4,50% |
| - Vitamine E | 0,02% |
| - Huile de silicone volatile | 5,00% |
| - Stéarate de PEG-40 (Myrj 52) | 2,00% |
| - Sodium PCA | 6,00% |
| - EDTA disodique | 0,05% |
| - Chlorure de strontium | 10,00% |
| - Glycérine | 3,00% |
| - conservateur | 0,30% |
| - Sepigel 305 | 1,00% |
| - Eau | qsp 100 % |

### Exemple 20 : Crème de soin pour dermatite atopique au cours des poussées eczémateuses

| | |
|---|---|
| - Palmitate d'éthyl hexyle | 3,00% |
| - Fraction liquide de beurre de karité (Triglycérides d'acide palmitique-stéarique-oléïque-linoléïque) | 2,00% |
| - Stéarate de glycérol | 3,00% |
| - Tristéarate de sorbitane (Span 65 de chez ICI) | 0,90% |
| - Polyisobutène hydrogéné | 4,50% |
| - Vitamine E | 0,02% |
| - Huile de silicone volatile | 5,00% |
| - Stéarate de PEG-40 (Myrj 52) | 2,00% |
| - Sodium PCA | 6,00% |
| - EDTA disodique | 0,05% |
| - Chlorure de strontium | 5,00% |
| - 17-valérate de bêta-méthasone (corticoïde) | 0,05% |
| - Glycérine | 3,00% |
| - conservateur | 0,30% |
| - Sepigel 305 | 1,00% |
| - Eau | qsp 100 % |

### Exemple 21 : Lotion de soin pour eczéma

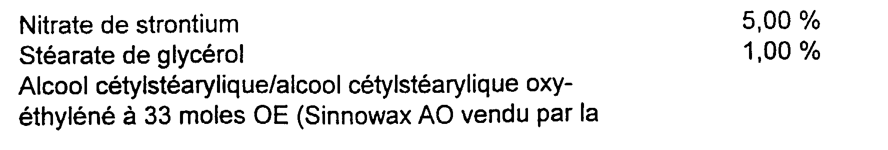

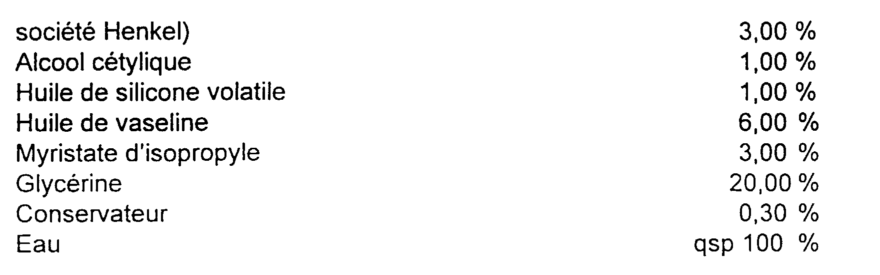

### Exemple 22 : Emulsion H/E destinée au traitement de l'acné

| | |
|---|---|
| *Phase grasse :* | |
| - Huile d'amandes d'abriccot | 14,50 % |
| - Fraction liquide de beurre de karité (Triglycérides d'acide palmitique-stéarique-oléïque-linoléïque) | 8;00 % |
| - Mono-stéarate de sorbitane (Span 60 de chez ICI) | 2,50 % |
| - Huile de purcellin | 2,00 % |
| | |
| *Phase aqueuse :* | |
| - Conservateurs | 0,50 % |
| - Sel disodique de l'acide éthylène di-amine tétracétique 2H₂O (complexant) | 0,05 % |
| - Neutralisant | 0,50 % |
| - Gélifiant | 0,70 % |
| - Glycérine | 5,00 % |
| - Monostéarate de sorbitane oxyéthyléné (20OE) (Tween 60 de ICI) (tensioactif) | 2,50 % |
| - Chlorure de strontium | 1,00 % |
| - Eau de la Roche-Posay | 60,00 % |
| - Eau déminéralisée ou permutée | qsp 100 % |

### Exemple 23 : Lait hydratant du visage

| | |
|---|---|
| Chlorure de strontium | 5,00 % |
| Stéarate de glycérol | 1,00 % |
| Alcool cétylstéarylique/alcool cétylstéarylique oxyéthyléné à 33 moles OE (Sinnowax AO vendu par la société Henkel) | 3,00 % |
| Alcool cétylique | 1,00 % |
| Polydiméthylsiloxane (DC 200 Fluid vendu par la société Dow Corning) | 1,00 % |
| Huile de vaseline | 6,00 % |
| Myristate d'isopropyle (Estol IPM 1514 vendu par Unichema) | 3,00 % |
| Antioxydant | 0,05 % |
| Glycérine | 20,00 % |
| Conservateur | 0,30 % |
| Eau | qsp 100; % |

### Exemple 24 : Crème de soin de l'érythème solaire pour peaux sensibles (émulsion H/E)

| | |
|---|---|
| Chlorure de strontium | 4,00 % |
| Stéarate de glycérol | 2,00 % |
| Polysorbate 60 (Tween 60 vendu par la société ICI) | 1,00 % |
| Acide stéarique | 1,40 % |
| Acide glycyrrhétinique | 2,00 % |
| Triéthanolamine | 0,70 % |
| Carbomer | 0,40 % |
| Fraction liquide du beurre de karité | 12,00 % |
| Huile de tournesol | 10,00 % |
| Antioxydant | 0,05 % |
| Parfum | 0,50 % |
| Conservateur | 0,30 % |
| Eau | qsp 100 % |

## Revendications

1. Utilisation d'au moins un sel d'au moins un métal alcalino-terreux comme inhibiteur de TNF-alpha, dans ou pour la fabrication d'une composition contenant un milieu cosmétique, pharmaceutique, dermatologique ou vétérinaire.

2. Utilisation d'au moins un sel d'au moins un métal alcalino-terreux comme inhibiteur de TNF-alpha, dans une composition contenant un milieu cosmétiquement, pharmaceutiquement ou dermatologiquement acceptable, pour traiter les peaux sensibles.

3. Utilisation d'au moins un sel d'au moins un métal alcalino-terreux dans ou pour la fabrication d'une composition cosmétique, pharmaceutique, dermatologique ou vétérinaire destinée au traitement des désordres pathologiques et/ou physiologiques associés à la libération de TNF-alpha.

4. Utilisation d'au moins un sel d'au moins un métal alcalino-terreux dans ou pour la fabrication d'une composition contenant un milieu cosmétiquement, pharmaceutiquement et/ou dermatologiquement acceptable, pour traiter les peaux sensibles et/ou les rougeurs cutanées d'origine neurogène.

5. Utilisation selon l'une des revendications précédentes d'au moins un sel pour prévenir et/ou lutter contre la rosacée et/ou l'érythème pudique, les irritations cutanées et/ou les dartres et/ou les érythèmes et/ou les sensations dysesthésiques et/ou les sensations d'échauffement et/ou les prurits de la peau et/ou des muqueuses.

6. Utilisation selon l'une des revendications précédentes, caractérisée en ce que le sel est choisi par les sels de baryum, calcium, magnésium, strontium et/ou de béryllium.

7. Utilisation selon l'une des revendications précédentes, caractérisée en ce que le sel est choisi parmi les sels de magnésium et de strontium.

8. Utilisation d'au moins un sel de strontium dans un milieu physiologiquement acceptable dans et/ou pour la fabrication d'une composition cosmétique, dermatologique, pharmaceutique et/ou vétérinaire destinée à traiter les désordres pathologiques et/ou physiologiques associés à la libération de substance P.

9. Utilisation d'au moins un sel de strontium dans un milieu physiologiquement acceptable dans et/ou pour la fabrication d'une composition cosmétique, dermatologique, pharmaceutique et/ou vétérinaire destinée à traiter les affections cutanées, les affections bronchopulmonaires, les algies, les affections gastro-intestinales et/ou les maladies du système nerveux central.

10. Utilisation d'au moins un sel de strontium dans un milieu physiologiquement acceptable dans et/ou pour la fabrication d'une composition cosmétique, dermatologique, pharmaceutique et/ou vétérinaire destinée à traiter la dermatite atopique, l'eczéma, le psoriasis, l'acné.

11. Utilisation selon l'une des revendications précédentes, caractérisée en ce que le sel est choisi parmi les chlorures, les carbonates, les borates, les nitrates, les acétates, les hydroxydes, les sulfates, les sels d'acides de fruits et les sels d'acides aminés.

12. Utilisation selon l'une des revendications précédentes, caractérisée en ce que le sel est un nitrate ou un chlorure.

13. Utilisation selon l'une de revendications précédentes, caractérisée en ce que le sel est le nitrate ou le chlorure de strontium.

14. Utilisation selon l'une des revendications précédentes, caractérisée en ce que le sel est utilisé en une quantité représentant de 0,01 à 30% du poids total de la composition.

15. Utilisation selon l'une des revendications précédentes, caractérisée en ce que le sel est utilisé en une quantité représentant de 0,01 à 20% et de préférence de 0,5 à 10% du poids total de la composition.

16. Utilisation selon l'une des revendications précédentes, caractérisée en ce que la composition contient en outre au moins une eau minérale et/ou thermale.

17. Utilisation selon l'une des revendications précédentes, caractérisée en ce que la composition se présente sous une forme appropriée pour une application topique, injectable ou ingérable.

18. Utilisation selon l'une des revendications précédentes, caractérisée en ce que le milieu est une solution aqueuse, huileuse ou hydroalcoolique, une émulsion eau-dans-huile, une émulsion huile-dans-eau, une microémulsion, un gel aqueux, un gel anhydre, un sérum, une dispersion de vésicules, de microcapsules ou de microparticules.

19. Utilisation selon l'une des revendications précédentes, caractérisée en ce que la composition contient au moins un agent différent desdits sels choisi parmi les antagonistes de neuropeptides, les antagonistes de médiateurs de l'inflammation, les agents antibactériens, antiparasitaires, antifongiques, anti-inflammatoires, antiseptiques, antiprurigineux, anesthésiques, antiviraux, kératolytiques, anti-radicaux libres, antiséborrhéiques, antipelliculaires, antiacnéiques, les corticoïdes et les agents modulant la différenciation et/ou la prolifération et/ou la pigmentation cutanée.

20. Utilisation selon la revendication précédente, caractérisée en ce que l'agent est choisi parmi les anesthésiques, les antiparasitaires, les anti-inflammatoires non stéroïdiens, les antagonistes réceptoriels de substance P, les antagonistes réceptoriels de CGRP, les antagonistes d'interleukine 1, et les antagonistes d'histamine.

21. Utilisation selon la revendication 20 ou 21, caractérisée en ce que l'agent est utilisé en une quantité représentant de 0,000001 à 10% du poids total de la composition.

22. Utilisation selon l'une des revendications précédentes, caractérisée en ce que la composition contient en outre au moins un actif choisi parmi les protéines ou les hydrolysats de protéine, les acides aminés, les polyols, l'urée, les sucres et les dérivés de sucre, les vitamines, l'amidon, les extraits végétaux, les acides gras essentiels, les céramides, les huiles essentielles, les hydroxyacides.

23. Utilisation selon l'une des revendications précédentes, caractérisée en ce que la composition se présente sous une forme appropriée pour une application topique.

24. Utilisation selon l'une des revendications précédentes, caracatérisée en ce que la composition contient, en outre, au moins un adjuvant choisi parmi les corps gras, les silicones, les gélifiants hydrophiles, les gélifiants lipophiles, les conservateurs, les parfums, les antioxydants, les solvants, les charges, les filtres, les absorbeurs d'odeur, les matières colorantes.

25. Procédé cosmétique pour inhiber la fixation et/ou la synthèse et/ou la libération de TNF-alpha dans la peau, caractérisé en ce qu'on applique sur la peau, sur le cuir chevelu et/ou sur les muqueuses, une composition contenant au moins un sel d'au moins un métal alcalino-terreux dans un milieu cosmétiquement acceptable.

26. Procédé de traitement cosmétique des peaux sensibles et/ou des rougeurs cutanées d'origine neurogène, caractérisé en ce que l'on applique sur la peau, sur le cuir chevelu et/ou sur les muqueuses sensibles et/ou rosacés une composition contenant au moins un sel d'au moins un métal alcalino-terreux dans un milieu cosmétiquement acceptable.

27. Procédé de traitement cosmétique des symptômes prurigineux des désordres et/ou maladies de la peau et/ou des muqueuses, caractérisé en ce qu'on applique sur la peau, y compris le cuir chevelu, et/ou sur les muqueuses une composition contenant au moins un sel d'au moins un métal alcalino-terreux dans un milieu cosmétiquement acceptable.

28. Procédé selon la revendication 25 à 27, caractérisé en ce que le sel de métal alcalino-terreux est choisi parmi les chlorures, les carbonates, les borates, les nitrates, les acétates, les hydroxydes, les sulfates, les sels d'acides de fruits et les sels d'acides aminés.

29. Procédé selon les revendications 25 à 28, caractérisé en ce que le sel est choisi parmi les sels de calcium, de magnésium, de strontium, de baryum et/ou de béryllium.

30. Procédé selon l'une des revendications 25 à 29, caractérisé en ce que le sel est un sel de magnésium ou de strontium.

31. Procédé de traitement cosmétique des désordres physiologiques associés à la libération de substance P, caractérisé en ce qu'on applique sur la peau, le cuir chevelu et/ou les muqueuses, une composition contenant au moins un sel de strontium dans un milieu cosmétiquement acceptable.

32. Procédé selon l'une des revendications 25 à 31 caractérisé en ce que le sel est utilisé en une quantité représentant de 0,001 à 30% du poids total de la composition.

33. Procédé selon l'une des revendications 25 à 32, caractérisé en ce que le sel de métal alcalino-terreux est utilisé en une quantité représentant de 0,01 à 20% et de préférence de 0,5 à 10% du poids total de la composition.

34. Composition cosmétique, dermatologique, vétérinaire et/ou pharmaceutique, contenant dans un milieu cosmétique, dermatologique, vétérinaire et/ou pharmaceutique, au moins un sel d'au moins un métal alcalino-terreux et au moins un antagoniste de neuropeptide et/ou au moins un antagoniste de médiateur de l'inflammation, différent dudit sel.

35. Composition selon la revendication 34, caractérisée en ce que le sel est choisi parmi les chlorures, les carbonates, les borates, les nitrates, les acétates, les hydroxydes, les sulfates, les sels d'acides de fruits et les sels d'acides aminés.

36. Composition selon l'une des revendications 34 ou 35, caractérisée en ce que le sel est choisi parmi les sels de calcium, de magnésium, de strontium, de beryllium, et/ou de baryum.

37. Composition selon l'une des revendications 34 à 36, caractérisée en ce que le sel est un sel de magnésium ou de strontium.

38. Composition selon l'une des revendicaitons 34 à 37, caractérisée en ce que le sel est le chlorure ou nitrate de strontium.

39. Composition selon l'une des revendications 34 à 38, caractérisée en ce que l'antagoniste de neuropeptide est choisi parmi les antagonistes réceptoriels de substance P, les antagonistes réceptoriels de CGRP, les antagonistes d'interleukine 1.

40. Composition selon l'une des revendications 34 à 39, caractérisée en ce que l'antagoniste de neuropeptide ou de médiateur de l'inflammation est utilisé en une quantité représentant de 0,000001 à 10 % du poids total de la composition.

41. Composition selon l'une des revendications 34 à 40, caractérisée en ce que le sel de métal alcalino-terreux est utilisé en une quantité représentant de 0,01 à 30 % du poids total de la composition.

42. Composition selon l'une des revendications 34 à 41, caractérisée en ce que le sel de métal alcalino-terreux est utilisé en une quantité représentant de 0,01 à 20 % et de préférence de 0,5 à 10% du poids total de la composition.

43. Composition selon l'une des revendications 34 à 42, caractérisée en ce qu'elle contient en outre au moins un agent différent desdits sels choisi parmi les agents antibactériens, antiparasitaires, antifongiques, anti-inflammatoires, antiseptiques, antiprurigineux, anesthésiques, antiviraux, kératolytiques, anti-radicaux libres, antiséborrhéiques, antipelliculaires, antiacnéiques, les corticoïdes et les agents modulant la différenciation et/ou la prolifération et/ou la pigmentation cutanée.

44. Composition selon l'une des revendications 34 à 43, caractérisée en ce que le milieu cosmétiquement et/ou dermatologiquement acceptable est une solution aqueuse, huileuse ou hydroalcoolique, une émulsion eau-dans-huile, une émulsion huile-dans-eau, une microémulsion, un gel aqueux, un gel anhydre, un sérum, une dispersion de vésicules, de microcapsules ou de microparticules.

45. Composition selon l'une des revendications 34 à 44, caractérisée en ce que le milieu contient, en outre, au moins une eau minérale et/ou thermale.

46. Composition selon l'une des revendications 34 à 45, caractérisée en ce qu'elle contient au moins un actif choisi parmi les protéines ou les hydrolysats de protéine, les acides aminés, les polyols, l'urée, les sucres et les dérivés de sucre, les vitamines, l'amidon, les extraits végétaux, les acides gras essentiels, les céramides, les huiles essentielles, les hydroxyacides.

47. Composition selon l'une quelconque des revendications 34 à 46, caractérisée en ce qu'elle contient, en outre, au moins un adjuvant choisi parmi les corps gras, les silicones, les gélifiants hydrophiles, les gélifiants lipophiles, les conservateurs, les parfums, les antioxydants, les solvants, les charges, les filtres, les absorbeurs d'odeur, les matières colorantes.

48. Composition selon la revendication 47, caractérisée en ce que les gélifiants sont des dérivés cellulosiques.

49. Composition selon l'une des revendications 34 à 48, caractérisée en ce qu'elle est appropriée pour le nettoyage, le soin, la protection, le traitement, le maquillage, le démaquillage du visage, des mains, des pieds et/ou du corps humain.
